(19) Europäisches Patentamt
European Patent Office
Office européen des brevets

(11) **EP 3 252 156 B1**

(12) **EUROPEAN PATENT SPECIFICATION**

(45) Date of publication and mention
of the grant of the patent:
**28.07.2021 Bulletin 2021/30**

(51) Int Cl.:
***C12N 9/50*** *(2006.01)*

(21) Application number: **17174447.7**

(22) Date of filing: **05.06.2017**

(54) **METHOD FOR EXTRACTION AND/OR ISOLATION OF BROMELAIN FROM PINEAPPLE**

VERFAHREN ZUR EXTRAKTION UND/ODER ISOLIERUNG VON BROMELAIN AUS ANANAS

PROCÉDÉ POUR L'EXTRACTION ET/OU L'ISOLEMENT DE LA BROMÉLINE À PARTIR D'ANANAS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.06.2016 PT 109425**

(43) Date of publication of application:
**06.12.2017 Bulletin 2017/49**

(73) Proprietor: **UNIVERSIDADE CATOLICA
PORTUGUESA**
**4200-072 Porto (PT)**

(72) Inventor: **MARIA MANUELA, ESTEVEZ PINTADO**
**4200-072 PORTO (PT)**

(74) Representative: **Patentree**
**Edificio Net**
**Rua de Salazares, 842**
**4149-002 Porto (PT)**

(56) References cited:
**US-A- 3 455 787**

- DEVAKATE R V ET AL: "Purification and drying of bromelain", SEPARATION AND PURIFICATION TECHNOLOGY, ELSEVIER SCIENCE, AMSTERDAM, NL, vol. 64, no. 3, 12 January 2009 (2009-01-12), pages 259-264, XP025799637, ISSN: 1383-5866, DOI: 10.1016/J.SEPPUR.2008.09.012 [retrieved on 2008-10-19]

- ZATUL IFFAH MOHD ARSHAD ET AL: "Bromelain: an overview of industrial application and purification strategies", APPLIED MICROBIOLOGY AND BIOTECHNOLOGY, vol. 98, no. 17, 26 June 2014 (2014-06-26) , pages 7283-7297, XP055418184, DE ISSN: 0175-7598, DOI: 10.1007/s00253-014-5889-y

- CAMPOS DÉBORA A ET AL: "Platform design for extraction and isolation of Bromelain: Complex formation and precipitation with carrageenan", PROCESS BIOCHEMISTRY, ELSEVIER LTD, GB, vol. 54, 23 December 2016 (2016-12-23), pages 156-161, XP029934394, ISSN: 1359-5113, DOI: 10.1016/J.PROCBIO.2016.12.014

- Débora A Campos ET AL: "Biological extraction of bromelain from pineapple byproducts", Industrial Crops and Products Produção Aplicações e Mercado Biochemistry LWT Food Science and Technology, 1 December 2015 (2015-12-01), pages 163-168, XP55538274, Retrieved from the Internet: URL:http://repositorium.sdum.uminho.pt/bit stream/1822/41272/1/document_37873_1.pdf [retrieved on 2019-01-04]

- B. G. SMITH ET AL: "Polysaccharide Composition of Unlignified Cell Walls of Pineapple [Ananas comosus (L.) Merr.] Fruit", PLANT PHYSIOLOGY, vol. 107, no. 4, 1 April 1995 (1995-04-01) , pages 1399-1409, XP55538272, Rockville, Md, USA ISSN: 0032-0889, DOI: 10.1104/pp.107.4.1399

- C.B. FABIAN ET AL: "Precipitation of rice bran protein using carrageenan and alginate", LWT-FOOD SCIENCE AND TECHNOLOGY, vol. 43, no. 2, 1 March 2010 (2010-03-01), pages 375-379, XP055538302, United Kingdom ISSN: 0023-6438, DOI: 10.1016/j.lwt.2009.08.005

## Description

### Technical field

The present subject-matter relates to a method for extracting and separating bromelain from pineapple and/or pineapple peels and/or pineapple stems, which comprises a precipitation, in particular a biological precipitation, comprising a polysaccharide as a precipitant.

### Background

Several techniques are used nowadays to isolate and purify enzymes, but most of them are obsolete, such as chemical precipitation and extraction using organic solvents, producing extracts at high costs and low purity rate (Soares, Vaz, Correia, Pessoa, & Carneiro-da-Cunha, 2012). In many biotechnological industries (food, medical and pharmaceutical) the selective separation of an enzyme from fermentation broths, animal and vegetal sources has been a primary research interest for downstream processing operations (Desai, 2000). The extraction of an enzyme from a crude extract could be very difficult due to low protein concentration, among contaminants with similar physical properties in the same solution (Bart & Pilz, 2011; Hatti-Kaul & Mattiasson, 2003). Above of all, until now it was difficult development an extraction process that favors the both economic and technical aspects.

Bromelain (BR) is a generic name given to proteolytic enzymes found in vegetable tissues such as peel, stem, fruit and leaves of the Bromeliaceae family, including pineapple stem (Ananas comosus). It is usually distinguished as either fruit BR (EC 3.4.22.33) or stem BR (EC 3.4.22.32) depending on its source, with all commercially available BR being derived from the stem. Stem and fruit BR have a molecular weight of 33 kDa and 28 kDa with an isoelectric point of 9.5 and 4.6, respectively (Harrach et al., 1995). The extract of BR has been shown to exhibit its activity over a pH range of 4.5-9.8.

This protease has wide range of applications in food and pharmaceutical industries. Recently, a wide range of therapeutic benefits have been attributed to BR such as the reversible inhibition of platelet aggregation, relief from bronchitis, improved recovery after surgical traumas, and the enhanced absorption of drugs, particularly of antibiotics (Amid, Ismail, Yusof, & Salleh, 2011). One of the important pharmaceutical applications of BR is the enzymatic debridement of necrotic tissues from ulcers and burn wounds, as well as, medical treatment of cancer patients. In the food industry, has been used as a meat tenderizing enzyme, but also is used in brewing and functional protein at the pre-digestion, digestive aids and in pharmaceutical industry has been used as antithrombotic, treatment of osteoarthritis and absorption promoter of antibiotic drugs (Amid et al., 2011; Soares et al., 2012).

Document DEVAKATE R V ET AL, "Purification and drying of bromelain", SEPARATION AND PURIFICATION TECHNOLOGY, ELSEVIER SCIENCE, AMSTERDAM, NL, vol. 64, no. 3, relates to the purification of bromelain by precipitation and by chromatography.

Document US3455787 describes a process for producing bromelain from the stems of pineapple plants which includes grinding the stems and removing the liquid therefrom by pressing, and centrifuging the liquid to precipitate fine ice crystals following which the remaining liquid can be centrifuged. Preferably precipitants (organic solvents, salts) and a detergent soluble in the precipitant are present during cooling.

Document ZATUL IFFAH MOHD ARSHAD ET AL, "Bromelain: an overview of industrial application and purification strategies", APPLIED MICROBIOLOGY AND BIOTECHNOLOGY, DE, (20140626), vol. 98, no. 17, is an overview on the purification of bromelain from pineapple fruit which could be applied, such as, two-phase systems, reverse micellar extraction, ion exchange and adsorption.

Document Débora A Campos ET AL: "Biological extraction of bromelain from pineapple byproducts", Industrial Crops and Products Produção Aplicações e Mercado Biochemistry LWT Food Science and Technology, 1 December 2015, (2015-12-01), pages 163-168, discloses a method for extracting or separating bromelain from pineapple peels or pineapples cylinders, which comprises a precipitation with carrageenan as a precipitant.

Document B. G. SMITH ET AL: "Polysaccharide Composition of Unlignified Cell Walls of Pineapple [Ananas comosus (L.) Merr.] Fruit", PLANT PHYSIOLOGY, vol. 107, no. 4, 1 April 1995 (1995-04-01), pages 1399-1409, Rockville, Md, USA describes the polysaccharides of pineapple. It is a general characterization of pineapple and not an application of other polysaccharides.

Document C.B. FABIAN ET AL: "Precipitation of rice bran protein using carrageenan and alginate", LWT- FOOD SCIENCE AND TECHNOLOGY, vol. 43, no. 2, 1 March 2010 (2010-03-01), pages 375-379, XP055538302, United Kingdom relates to the application of polysaccharides (alginate and kapa carrageenan) in the separation of rice bran proteins, from a purified solution, therefore the possible interferences were removed.

These facts are disclosed in order to illustrate the technical problem addressed by the present disclosure.

## General Description

The biological precipitation can result in concentration and purification of BR, with maintenance of activity, bringing as advantages easy scale-up, simple equipment and large number of alternative precipitants; some of them are inexpensive or used at a very low concentration. Polysaccharides are precipitants that can interact with proteins, forming soluble or non-soluble complexes, by changing medium conditions, such as pH or ionic strength, the protein can be released, keeping its structure as well as its biological activity.

The present subject-matter relates to a method for extracting and separating bromelain from pineapple and/or pineapple peels and/or pineapple stems, which comprises a biological precipitation step comprising 0.05-0.5 % w/v of a polysaccharide as a precipitant, , wherein the polysaccharide is ʟ-carrageenan, wherein the ʟ-carrageenan molecular weight is from 400-600 kDa, wherein pH of the precipitation is 4-6.

In an embodiment for improved results, the biological precipitation step, may comprise 0.05-0.3% w/v of a polysaccharide as a precipitant.

In the present disclose, it is considered that the precipitation is a biological precipitation because the precipitant agent is a polysaccharide.

In an embodiment for improve results, the polysaccharide molecular weight may be from 430-550 kDa.

In an embodiment for improve results, the pH of the precipitation may be pH 4.6-5.

In an embodiment for improve results, the method now disclosed may further comprise the steps of: obtaining a pineapple juice/pomace/pulp.

In an embodiment for improve results, a buffer is added to the precipitation, preferably acetate buffer.

In an embodiment, carrageenan is a family of polysaccharides obtained from certain species of red seaweeds (Rhodophyceae), non-toxic and water soluble. Carrageenans are hydrophilic linear sulphated galactans, mainly consist of alternating 3-linked β-D-galactopyranose (G-units) and 4-linked a-D-galactopyranose (D-units) or 4-linked 3,6-anhydro-a-D-galactopyranose (DA-units), forming disaccharide repeating unit of Carr. These polysaccharides are traditionally split into six basic forms, but the three commercial most important are Kappa (κ)-, Iota (ʟ)-, Lambda (λ) Carr, having one, two and three sulphate ester groups, respectively, corresponding to sulphate contents of 22%, 32% and 38% (w/w) (De Ruiter & Rudolph, 1997). Iota and kappa-Carr are gel-forming agents due a "crosslinking" spiral chains, double helix, with the sulphate groups oriented towards the external part (Morais, VALLE, & Pizzinatto, 1989).

## Brief Description of the Drawings

The following figures provide preferred embodiments for illustrating the description and should not be seen as limiting the scope of disclosure.

Figure 1: a) Titration of BR with increasing concentration of Carr, at different concentrations of NaCl (0, 100, 200 and 500 mM). b): turbidimetric evaluation of complex formation. Medium: 25 mM acetate buffer, pH 5.1. BR concentration of 3 mg/mL. Enzyme activity was measured at pH 5.1.

Figure 2: Recovery of BR and activity measurement in the precipitate and in the supernatant at different initial concentrations of Carr. Medium: 25 mM acetate buffer, pH 5.1. BR concentration of 3 mg/mL. Enzyme activity was measured at pH 5.1.

Figure 3: Right - Thermal stability of BR and its complex with Carr evaluated by fluorescence at 350 nm. Left - Evaluation of unfolding fraction of BR and its complex with Carr Medium: 25 mM acetate buffer, pH 5.1. BR at 3 mg/mL and Carr at 1.5 mg/mL.

Figure 4: Hydronamic diameter and intensity of Br-Carr complex formation in an acid-base titration at 0 mM NaCl. Critical pH at left of 3.97 and at right 6.75. Medium: 25 mM acetate buffer. BR concentration of 3 mg/mL and Carr at 1.5 mg/mL.

Figure 5: Acid-base titration of BR and BR-Carr complex at 0 mM NaCl and at 200 mM NaCl, evaluation of zeta potential (mV) by Dynamic light scattering. Medium: 25 mM acetate buffer, initial pH 4.5. BR concentration of 3 mg/mL and Carr at 1.5 mg/mL.

Figure 6: Recovery of BR and activity measurement in the precipitate and in the supernatant at different initial concentrations of Carr. Medium: aqueous extract of pineapple residues (stems (**a**), and peels (**b**)), pH in solution of 4.3. Enzyme activity was measured at pH 5. The circles represent the activity on the supernatant and the squares represent the activity recovery from the precipitate.

**Figure 7:** Experimental design encompassing the desirability of the optimized models for the two tested extracts (a) stems and (b) peels. Effect of each independent variable (i.e., BR concentration, Carr concentration and pH), pertaining to three responses (i.e., turbidity, relative activity of BR, total protein and specific activity).

**Figure 8:** Experimental design encompassing the effect of each independent variable (i.e., BR concentration, Carr concentration and pH), pertaining to three responses (i.e., turbidity, relative activity of BR, total protein and specific activity), obtained from stems of pineapple residues.

**Figure 9:** Experimental design encompassing the effect of each independent variable (i.e., BR concentration, Carr concentration and pH), pertaining to three responses (i.e., turbidity, relative activity of BR, total protein and specific activity), obtained from peels of pineapple residues.

## Detailed Description

The present subject-matter relates to a method for extracting and separating bromelain from pineapple and/or pineapple peels and/or pineapple stems, which comprises a biological precipitation comprising a polysaccharide as a precipitant.

In an embodiment, bromelain from the stem of Ananas comosus (BR), ᴸ-carrageenan (Carr), alpha-N-carbobenzyloxy-l-lysine p-nitro-phenyl ester (LNPE) and L-cysteine were purchased from Sigma-Aldrich (St. Louis, Missouri, USA), all the other reagents were of analytical quality.

In an embodiment, preparation of standard stem Bromelain: Standard BR was prepared at a concentration of 100 mg/mL in 100mM sodium acetate buffer, pH 4.6, 0.1 M EDTA and 0.3M KCl were added to prevent enzyme degradation and improvement enzyme solubility, as described by (Rocha & Nerli, 2013). The solution was kept at -18 °C. To check eventual loss of protein structural integrity with the storage, measurement of enzyme activity was carried out before using.

In an embodiment, determination of BR activity: BR activity was determined by using the substrate LNPE (Heinrikson, 1976). Briefly, the substrate was used in the assay at a final concentration of 0.23 mM in buffer 30 mM sodium acetate buffer, pH 4.6, improved with 100 mM KCl and with 1.0 mM of L-Cysteine. The extent of the enzymatic reaction, represented by the release of p-nitrophenol, was measured spectrophotometrically for 5 minutes at 340 nm, 25 °C and continuous stirring. One unit of enzymatic activity is the amount of BR that will release 1.0 mol of p-nitrophenol from LNPE in 1 minute under the experimental conditions.

In an embodiment, the determination of total protein concentration: It was carried out using the bicinchoninic assay (BCA) (Brown, Jarvis, & Hyland, 1989; Walker, 2009). A fresh standard working reagent (SWR) was prepared mixing 100 vol of reagent A (bicinchoninic acid solution; Sigma-Aldrich, St. Louis, Missouri, USA) with 2 vol of reagent B (CuSO4 solution 4% (w/v) prepared from $CuSO_4.5H_2O$; Sigma-Aldrich, St. Louis, Missouri, USA). A volume of 50 μL of protein solution (maximum concentration of 1 mg/mL) was added to 1 mL of SWR. The tubes were incubated at 37 °C for 30 min. After leaving them to cool down at room temperature, the absorbance was measured at 562 nm using a cell with a 1 cm path length. The calibration curve was performed using dilutions of a standard solution of bovine serum albumin 1 mg/mL.

In an embodiment, the solubility diagram of BR-Carr complex: Turbidity (absorbance at 420 nm) of solutions of 3 mg/mL of BR with 0.1% (w/v) of Carr was measured and plotted against pH. The pH variations of the medium were obtained by adding NaOH or HCl aliquots and leaving the pH of the system to equilibrate before measuring the turbidity. These titration curves were made in order to estimate the pH range where the protein-polymer complex is soluble or non-soluble (Heinrikson, 1976; Valetti et al., 2012).

In an embodiment, turbidimetric titration curves with Carr: The formation of the insoluble BR-Carr complex was monitored by means of turbidimetric titration. A fixed BR concentration (3 mg/mL) in 30 mM acid acetic/acetate buffer pH 4.6 was titrated at 25°C in a glass cell using Carr solution as titrant. To avoid changes in pH during titration, both BR and Carr solutions were adjusted to the same pH value. The absorbance of solution at 420 nm was used to follow the BR-Carr complex formation and plotted vs. the total Carr concentration in the tube. The results were fitted with a 4-parameters sigmoidal function in order to determine the value of the Carr minimal concentration required to precipitate BR. This parameter was calculated as the intersection of the tangent at the inflection point with the plateau of the plot. The [BR]/[Carr] ratio can be calculated as the ratio between the BR total concentration and the [Carr] calculated. Absorbance solutions were measured using a Jasco 520 spectrophotometer (Jasco Analytical Instruments, Washington, USA) with constant agitation in a thermostatized cell of 1 cm of path length (Heinrikson, 1976).

In an embodiment, the Zeta potential and size of the complexes particles: A Zetasizer Nano ZSP (Malvern Instruments, Worcestershire, UK) using dynamic light scattering (DLS) was used to measure the particle size (PS) and $\xi$-potential (ZP).The complex BR-Carr stock solutions were produce in 30 mM acetate buffer, the pH was adjusted by adding aliquots of NaOH for pH increasing and HCl to decreasing pH, varying from 9.0 to 1.0 by 0.5 unit increments with a confidence interval of $\pm$ 0.1 unit. Particle sizes (hydrodynamic radius) were measured by taking into account the first order result from a DLS experiment as an intensity distribution of PS. The intensity distribution was weighted according to the scattering intensity of each particle fraction or family. Data were validated only if the cumulant fit error was <0.005. $\xi$-potential was measured using Laser Doppler Anemometry (LDA). All analyses were carried out three times and sample readings were done in triplicate with an angle of 90° at 25 °C.

In an embodiment, the thermal stability of BR and BR-Carr complex: The thermal stability of BR and complex BR-Carr was measured by the native fluorescence emission of the protein at 350 nm. The samples were exposed to a temperature gradual increasing between 25-90 °C, every 5 °C were taken samples and the BR activity and the relative fluorescence emission were measured. The data analysis was made assuming an approximation of two-state model of denaturation (N: native and D: denatured) where only the native and unfolded states were significantly populated.

Being K, the equilibrium constant, it can be written as:

$$K = \frac{[D]}{[N]} \quad (1)$$

The unfolded protein fraction was calculated as:

$$\alpha = \frac{F_i - F_D}{F_N - F_D} \quad (2)$$

Where: $\alpha$ is the unfolded protein fraction, FN and FD are the fluorescence emission of the native and unfolded states respectively, Fi is the fluorescence at any temperature. We have used non-linear least squares to fit the unfolded protein fraction versus temperature data, and the temperature at the mid-point of denaturation (Tm) was determined. The equilibrium constant, K, for the unfolding process can be calculated:

$$K = \frac{\alpha}{1-\alpha} \quad (3)$$

From the following equation the free energy ($\Delta$G°) of the denaturation process can be calculated as:

$$\Delta G° = -RT \ln K \quad (4)$$

And the enthalpy change ($\Delta$H°) from equation:

$$\frac{\partial \ln K}{\partial T} = -\frac{\Delta H}{T} \quad (5)$$

Finally, the entropic change ($\Delta$S°) of the unfolding process vs temperature can be calculated as:

$$\Delta G = \Delta H - T \, \Delta S \quad (6)$$

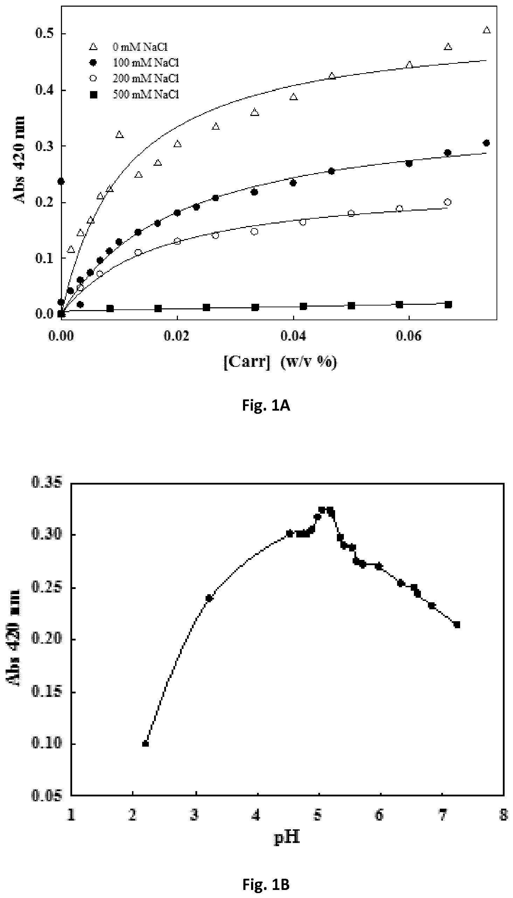

In an embodiment, the solubility phase diagrams of Carr-BR complex were determined: complex formation between Carr and BR as function of pH was determined as shown at the **Fig. 1B**).

In an embodiment, the formation of a complex was observed (a constant protein/ polymer ratio) to be influenced by the pH of medium. The increase in pH above 5.0 induced an increasing in the turbidity, with a maximum at pH 5.1. At higher pH values the turbidity decreased in agree with the solubilisation of the non-soluble complex. Carr has negative electrical charge from pH 2 due to the presence of strong acidic groups (sulphonic), while BR (isoelectrical pH 9.5) has positively electrical charge in acid pH, the positive electrical change of the BR decreases in the sense that the pH is increasing, a diminution of the turbidity was observed due a minor formation of BR-Carr complex. In the acid range of the curve (left) the decrease in the turbidity should be due to partial protonation of some sulphonic groups of Carr which induces a loss of negative electrical charge density on the polymer chain (Valetti et al., 2012).

In the embodiment of **Figure 1** it is shown the variation of the medium absorbance at 420 nm when BR was titrated with Carr at pH 5.1 in presence of different ionic strength. The Abs values increased until remained a constant value (plateau) indicating a higher extent of complex formation. The salt presence increases the solubility of the complex, as it was observed by the decrease of the turbidity, being completely annulling at 500 mM of NaCl. Taking this into account, the interaction of BR with Carr seems to be only of columbic in nature. From these curves, it was possible to calculate the minimal optimal Carr amount needed to precipitate BR, which correspond to the case in which most BR has been precipitated as an insoluble complex, from the fitting data of **Fig. 1** the stoichiometry ratio was 3.0 mg protein/ 0.01 mg polymer (in an embodiment, the Carr concentration necessary to precipitate the BR around 0.001% w/v, preferably around 0.005% w/v). This finding shows that the Carr concentration needed to precipitate BR was very low, when compared to other traditional proteins precipitant methods, using inorganic cations and anions, demonstrating a high potential for applications of the method in scaling up. In many cases, the target proteins are present in high volumes of solution, but even so, a small mass of this polyelectrolyte is important to precipitate the enzyme.

In an embodiment, a complementary experiment to confirm that Carr presence does not modify the biological activity of BR was performed. A constant concentration of BR was incubated in media of increasing Carr concentration, in particular at pH 4.6, during in particular 30 minutes, and then the non-soluble complex was separated by centrifugation and re-dissolved in Tris-HCl buffer, pH 8.2 with addition of 500 mM of NaCl.

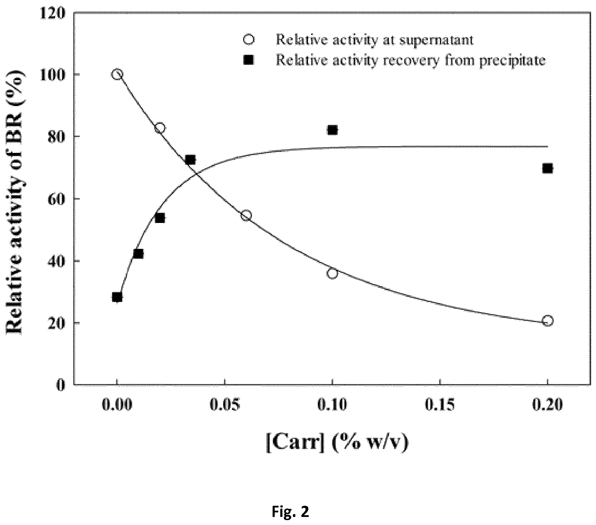

In an embodiment, the enzymatic activity of BR was determined in the supernatant and in the re-dissolved precipitate, the results are shown in **Fig 2.** Around 80% of the activity was recovered, in the solubilized precipitated; while 20% of the activity remained in the supernatant in agree that the following equilibrium is displaced in a great part to non-soluble complex formation:

$$BR\,(aq) + Carr\,(aq) \leftrightarrow BR - Carr\,(pp)\,(7)$$

In an embodiment, this experiment validates the viability of this method as separation way of BR.

In an embodiment, the emission of the native fluorescence of BR in the presence and absence of Carr was carried out as follows: fluorescence emission of proteins is dominated by tryptophan, which absorbs at the longest wavelength (in water, 350 nm). The effect of Carr on protein structure was monitored using fluorescence spectroscopy (Lakowicz, 1994). The emission spectra of BR in buffer acetate pH 5.1 in the absence and the presence of Carr (0.034% w/v) were obtained (data not shown). The presence of Carr does not affect the fluorescence spectrum, which means that the polysaccharide did not change the microenvironment of tryptophan residues presents in BR.

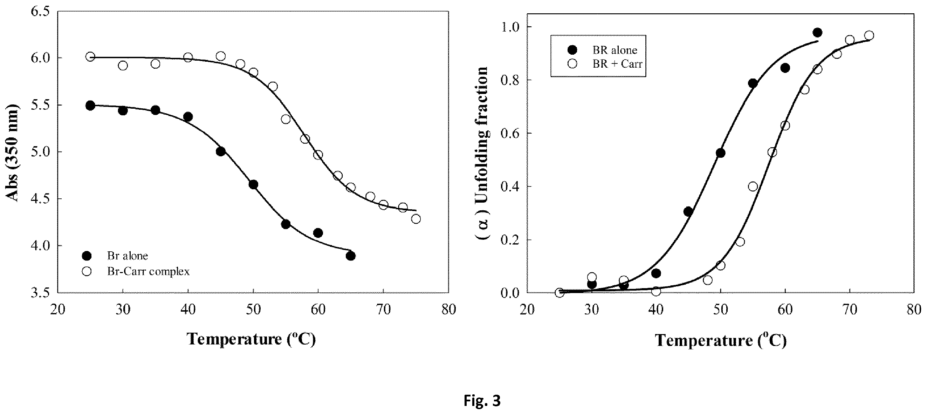

In an embodiment, the thermal stability of the BR in the Carr presence was performed as follows: the effect of the temperature on the enzyme in the absence and presence of Carr was assayed by measuring the native fluorescence emission of BR at 350 nm vs. temperature as shown at **Fig. 3.** The thermal pattern of **Fig. 3** shows that the presence of Carr induces a thermodynamic stabilization of BR since an increase in the $T_m$ values was observed as shown at Table I. These results are encouraging since BR seems to be stable over significant increment of temperature in medium with Carr. This fact comes unsurprisingly, since it is known that the addition of co-solutes such as polysaccharides and other hydrophilic substances to a protein solution leads to an enhanced structural stability.

**Table I:** Thermodynamic thermal stability parameters of BR and its complex. Medium: 25 mM acetate buffer, pH 5.1. Enzyme concentration 3 mg/ml and polyssacharide at 1.5 mg/ml.

| System | Tm (°C) | $\Delta$H° (Kcal/mol) | $\Delta$S° (e.u.) | $(\partial\alpha/\partial T)_{0.5}$ |
|---|---|---|---|---|
| **BR alone** | 49.3 $\pm$ 0.8 | 13.9 | 43.1 | 4.48 |
| **BR-Carr** | 57.8 $\pm$ 0.4 | 50.8 | 153.0 | 3.67 |

Table I show the calculated values of $\Delta H$ and $\Delta S$ for BR unfolding in absence and presence of Carr. The entropic and entalphic changes were positive consistent with the published results for protein denaturation. The inflection point of the thermal unfolding curves $((\partial\alpha/\partial T)_{0.5})$ gives an idea of the cooperativity between protein-polysaccharide at the unfolding process and how the polysaccharide presence changes this process. The values were calculated as explain above and are described at Table I.

In an embodiment, it can be seen that the polymer presence induced a decrease in the cooperative factor suggesting a diminution in the response of the enzyme to the thermal change by the Carr presence.

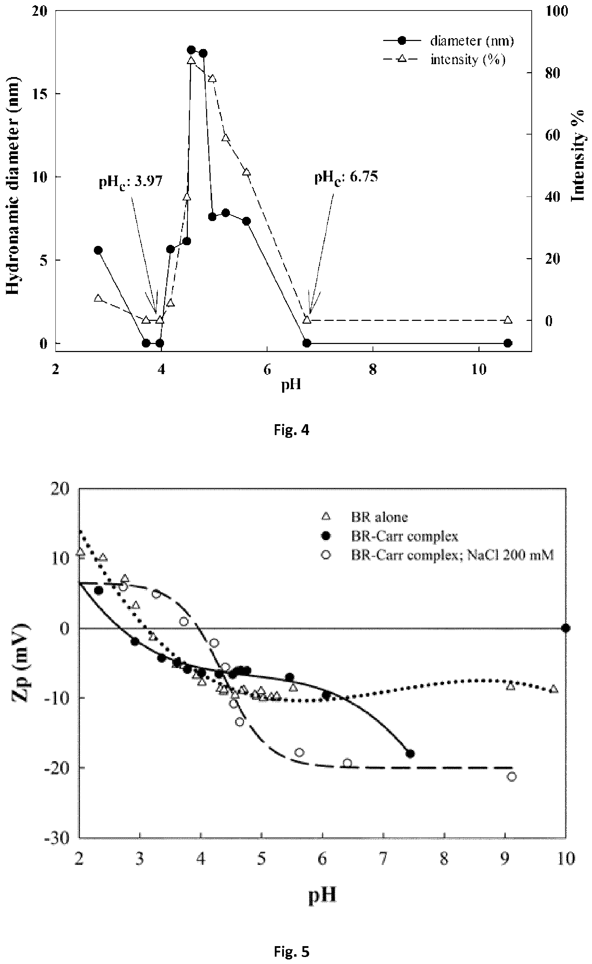

In an embodiment, the effect of pH on size and zeta potential of BR in the absence and presence of Carr was performed. The **Fig. 4** shows the pH effect at the complex formation of BR in presence of Carr. The Hd was evaluated, as well as, the intensity percentage of complexes formation. A Hd pick of 17.5 $\pm$ 0.1 nm and intensity pick of 84% was achieved for a pH between 4.6 and 4.8, this value converted at molecular weight correspond to 541 $\pm$ 12 kDa, which was indicative of complexes formation, since BR alone has a theoretical value of 33 kDa and Carr between 400-600 kDa (polydisperse molecular weight), thus the Hd pick obtained was the protein-polysaccharide complex.

In an embodiment and for pH $\geq$ 5 it is observed a Hd group of ca. 7 nm (ca. 80 kDa) and for pH $\geq$ 6.7 the these Hd groups disappeared completely, these values keep up with intensity, indicating the complete dissolution of complex. On the other hand, for pH $\leq$ 4.5 the Hd values decrease to ca. 6.0 $\pm$ 0.2 nm, corresponding to 35 $\pm$ 2 kDa, which shows the protein free in solution. It was clear, that pH modifies the complex formed in quantity and quality. Two critical pH (pHc) of complex formation were obtained with the acid-base titration curve, pH 3.97 and 6.75, being between these values the range of non-soluble complex formation. The complex formation involves the interaction between proteins and polysaccharides, and in some cases, even hundreds of proteins could be bonded to just one polysaccharide molecule. These connections at the complex could be unbonded by shifting pH and ionic strength of the medium.

In an embodiment, the formation of the soluble complex can be followed by light scattering measurements by measuring Zp (charges measure at the medium) shown at **Fig. 5** and the Hydronamic radius (Hd) as shown at **Fig. 4.** In a second step, an interaction is produced between the soluble complex particles, so, a non-soluble complex (of high molecular mass) is produced and this second step is easily followed by turbidimetry (measuring the absorbance of the medium at 420 nm, **Fig. 1B**). Finally, the non-soluble particles interact between them to form a macro-aggregate state complex (Boeris et al., 2013).

In an embodiment, **Fig. 5** shows the dependence of the Zp of BR in the absence and presence of Carr and NaCl 200 mM. It has been reported that stem and fruit BR have a molecular weight of 33 kDa and 28 kDa with an isoelectric point of 9.5 and 4.6, respectively. The extract of commercial BR used in this work is from stem, however, both BR are present. This can be observed from the data Zp vs pH (from **Fig. 5**) where the Zp value of BR-Carr complex decreased respect to the free BR, due to neutralization of the residual positive electrical charge of BR. However, in the presence of NaCl 200 mM, the Zp of the complex increased due to screening of the electrical charge by the salt.

In an embodiment, this was due to the interaction between soluble complexes that interacts with the medium at low electrical charge, so the attraction forces are major than the repulsion ones. In the sense, the increasing of pH does not increase the number of negative electrical charge of the Carr, but a lost of positive charges of BR and because of that the balance between the positive and negative charges were not compensated, so the complexes increase their negatively, which induces repulsion between the particles of complex of minor mass. As result, a diminution of the diameter of the complexes was observed, but they exist in a higher pH interval.

In an embodiment, the traditional methods for the BR isolation have been through chemical precipitation, using ammonium sulphate at 50%, acetone 80% and ethanol 60% (Chaurasiya & Hebbar, 2013; Soares et al., 2012). In these methods the recovery of BR activity is around 85% with a purification factor 3 to 4.90. However, the precipitation with ethanol induce a decrease in the alpha helices content of BR (Soares et al., 2012) and a significant decreasing in the native fluorescence emission in agree with a loss of the secondary and tertiary structure. The main problem of these methods is the use of high concentration of salt or solvent, those can be not discarded in the environment, so they must be recovery at the end of the process. In the case of ammonium sulphate the recovery of this high soluble salt is difficult, so an increase in the cost of the total process in produced.

In an embodiment, the use of natural polysaccharides electrically charged (polyelectrolytes) as precipitant agents by formation of non-soluble complexes, where the enzyme can be recovery by inversion of the conditions such as change of the medium pH or low salt concentration, is an advantage on the tradition methods. It was found that Carr does not modify the biological activity of the BR and increases the thermal stability of the enzyme around 8 °C. Was demonstrate at this research work that the natural polyelectrolyte Carr allow to precipitate BR under a non-soluble complex, which can be re-dissolved and recovery by change in a slightly manner medium variables, such as, the NaCl concentration and pH values. Also, was proved that BR maintained its biological activity (yield of activity was 80% at precipitate) through precipitation process, since Carr also acted as enzyme stabilizer and above of all is a nontoxic and the food grade polysaccharide. The complex formation and precipitation method showed be suitable for application at BR isolation method.

In an embodiment, bromelain, carrageenan and pH were modulated through a central composite design (CCD). The three independent variables were studied at three levels and coded as -1 (lowest level), 0 (central level), +1 (highest level). The complete design consisted of 16 experimental trials, including two replicates of the central point. Each of the 16 systems was evaluated in triplicate. Response variables were estimated using the response surface model described by the following second-order polynomial equation.

In an embodiment, the variables present in linear terms represent the coordinates of the maximum value predicted, those in quadratic terms represent the surface curvature, and the bi-factorial cross-products represent the directions of the axes of the geometric figure obtained by sectioning the surface area. In order to verify the adequacy of the fitted model, analysis of variance and test of lack of fit were performed.

In an embodiment, the response surface methodology was used to analyse the effect of the combinations of the three independent variables on complex formation evaluated by turbidimetry, Br activity and total protein after precipitation and redissolution of complex. A predictive model was used to graphically represent the systems.

In an embodiment, the multi-criterion response surface optimization based on the Derringer's desirability function was employed (Suich and Derringer 1980). The function transforms the response of each variable into a desirability score (d) ranging between 0 (completely undesirable) and 5 (entirely desirable). The function takes different forms depending on the used optimization criterion: maximization, minimization, or attaining a fixed target.

In an embodiment, all fits, and associated statistical analyses related with the experimental design were performed using StatGraphics centurion version 16.1.

In an embodiment, the experimental design at the response variables obtained a $R^2$ around 98%, demonstrating the adequacy of the model to the group of results presented at 95% confidence level. Taking into account the response variable in this study we conclude that the optimum and maximum conditions for precipitation of BR with carrageenan were: 3 mg/ml of Br to 0. 1 mg/ml of carrageenan to an optimal pH of 5.0, preferably 3 mg/ml of Br to 0.01 mg/ml of carrageenan to an optimal pH of 5.0.

Thus, was developed a new mining model for extraction, isolation and purification of Bromelain using low-cost technique, which can be easily applied to industry, with easy scale-up and the method can use industrial wastes for recovery of Bromelain.

In an embodiment, the precipitation of BR was studied using pineapple residues extracts (stem and peels). Thus, a constant concentration of each extract was incubated with increasing Carr concentration, at pH 4.6 and then the insoluble complexes were separated by centrifugation and redissolved in Tris-HCl buffer, pH 8.2 with the addition of 500 mM of NaCl.

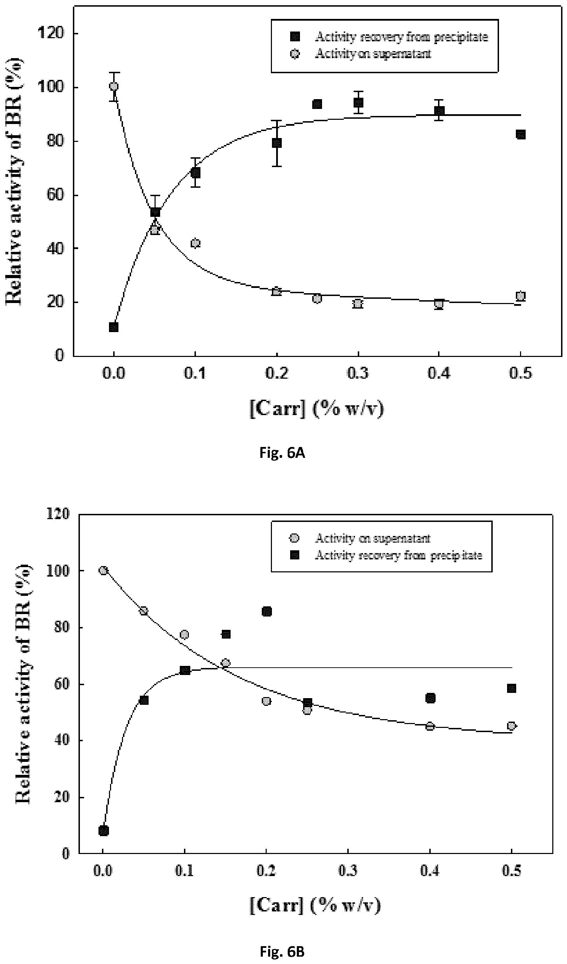

In an embodiment, the enzymatic activity of BR was determined in the supernatant and precipitate; the results are shown in **Fig. 6.**

In an embodiment, for stem extract only a low concentration of Carr (0.05%, w/v) was needed to displace 50% of available BR to the precipitate, also in average ca. 90% of the activity was recovered, in the solubilized precipitate for 0.3% of Carr (w/v), while 10% of the activity remained in the supernatant confirming that the equilibrium was displaced to a large extent to insoluble complex formation. When studying, peel extracts a higher Carr (0.1%, w/v) concentration was needed to observe a 50% passage of the available BR to insoluble complex. However, only 0.2% (w/v) of Carr was needed to transfer ≥80% of available BR to the precipitate, while ca. 10% of activity remained in the supernatant. When testing higher concentrations of Carr (≥0.25%, w/v), it was observed that the supernatant activity was maintained but drastically decreased on precipitate.

In an embodiment and in order to create a model for each extract, the BR was quantified by HPLC methods, as described above. The stem and peel extracts presented a BR concentration of ca. 70 mg/mL and ca. 85 mg/mL, respectively. For the design of the extraction model, a high concentration of extract was established to mimetize a possible application at industrial scale. The concentrations were adjusted for each model, stem extract between 1.57 and 2.28% (w/v) and peel extract between 2.1 and 2.8 (w/v).

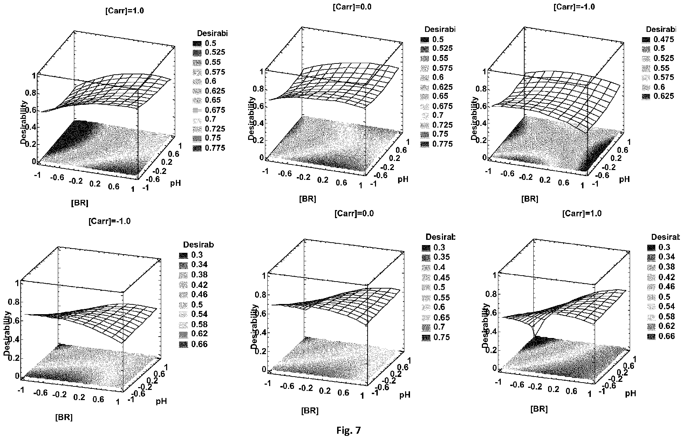

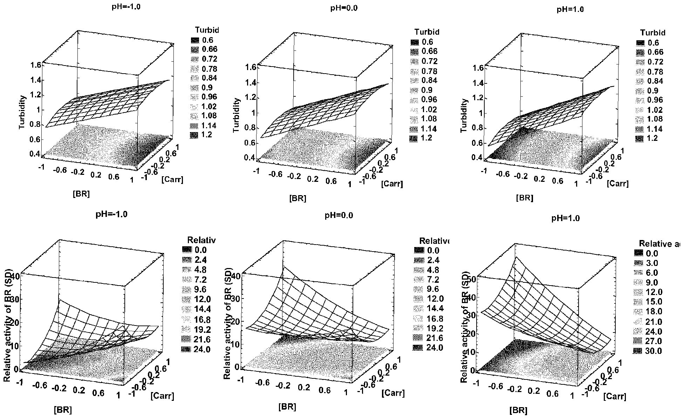

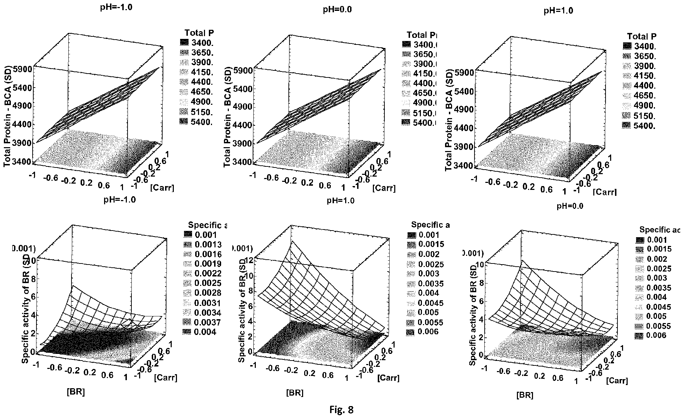

In an embodiment, to obtain an optimized model, desirability was calculated using the predicted model for stem extract, and the desirability index of the model was 72.59%. The responses showed that for observed data the more convenient result were those from experiment 3, but using the predict data from the adjusted model, the most suitable results were those from experiment 5 (coded responses, -1; 0; -1). This prediction shows the precipitation obtained nearest of the optimum values, but not the exact values of the prediction (coded responses, -0.306; -0.119; -0.999). When transporting the predict data to the un-coded values, it was possible to determine that the optimum values for BR isolation and recovery were 52 mg/mL of BR concentration, 172.5 $\mu$g/mL of Carr at pH of 9.4; these values are shown in **Fig. 7,** where it is possible to visualize the desirability of complex formation of each response. **Fig. 8** shows all the surface models designs for all independent variables as well its interactions, when studying each dependent variable.

In an embodiment, the optimum characteristics for peel extract of the model were determined by a desirability index of 80.00%, at **Fig. 7,** was possible to visualize the desirability of complex formation of each factor. The responses showed that for observed data the more convenient result were those from experiment 17, but using the predict data from the adjusted model, the most suitable results were those from execution 24 (coded responses, 0; 1; -1), on the other hand, the exact predict values were -0.068; -0.021; -0.999 (coded responses), the un-coded responses represents 69.4 mg/mL de BR, 172.5 $\mu$g/mL of Carr at pH 9.4.

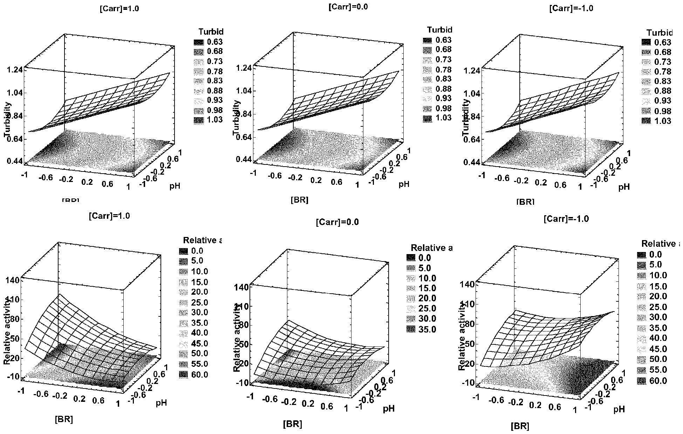

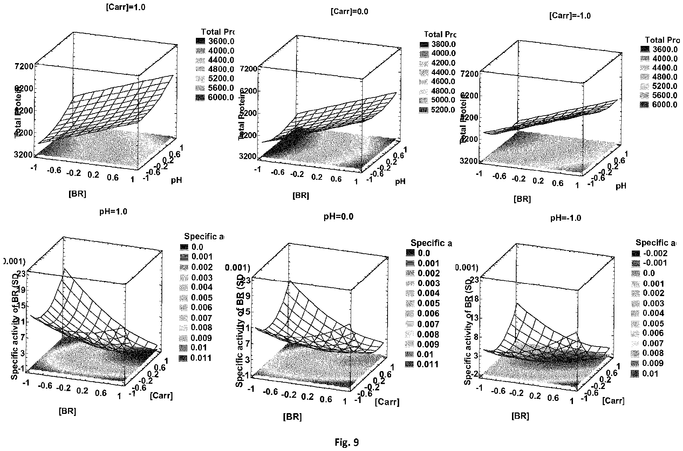

In an embodiment, **Fig. 9** shows all the surface models designs of each independent variables as well its interactions, when studying each dependent variable.

**Table II -** pH conditions

| | BR amount (% w/v) | pH | Precipitant (% w/v) | Yield (%) |
|---|---|---|---|---|
| **Stem pineapple extract** | 1.82 | 9.4 | 0.0173% | 90 |

(continued)

| | BR amount (% w/v) | pH | Precipitant (% w/v) | Yield (%) |
|---|---|---|---|---|
| **Peel pineapple extract** | 2.4 | 9.4 | 0.0173% | 80 |

**Table III -** Amount of polysaccharide

| | Polysaccharide optimum range (% w/v) | Minimal limits of polysaccharide required (% w/v) | Maximal limits of polysaccharide required (% w/v) |
|---|---|---|---|
| Commercial bromelain | 0.01 | 0.001 | 0.2 |
| Stem extract | 0.0173 | 0.005 | 0.6 |
| Peel extract | 0.0173 | 0.001 | 0.3 |

The term "comprising" whenever used in this document is intended to indicate the presence of stated features, integers, steps, components, but not to preclude the presence or addition of one or more other features, integers, steps, components or groups thereof.

## Claims

1.  A method for extracting or separating bromelain from pineapple and/or pineapple peels and/or pineapple stems, which comprises a precipitation, comprising 0.05-0.5% w/v of a polysaccharide as a precipitant, wherein the polysaccharide is ʟ-carrageenan, wherein the ʟ-carrageenan molecular weight is from 400-600 kDa, wherein pH of the precipitation is 4-6.

2.  The method according to any one of the previous claims wherein the pH of the precipitation is 4.6-5.

## Patentansprüche

1.  Ein Verfahren zur Extraktion oder Abtrennung von Bromelain aus Ananas und/oder Ananasschalen und/oder Ananasfruchtstandachsen, umfassend eine Fällung, umfassend 0,05-0,5 % w/v eines Polysaccharids als Fällungsmittel, wobei das Polysaccharid ʟ-Carrageenan ist, wobei das Molekulargewicht des ʟ-Carrageenans 400-600 kDa beträgt, wobei der pH-Wert der Fällung 4-6 ist.

2.  Das Verfahren nach einem der vorhergehenden Ansprüche, wobei der pH-Wert der Fällung 4,6-5 ist.

## Revendications

1.  Un procédé pour extraire ou séparer de la broméline à partir d'ananas et/ou de pelures d'ananas et/ou de tiges d'ananas, qui comprend une précipitation, comprenant 0,05-0,5% p/v d'un polysaccharide en tant que précipitant, dans lequel le polysaccharide est un ʟ-carraghénane, dans lequel le poids molléculaire du ʟ-carraghénane est de 400-600 kDa, dans lequel le pH de la précipitation est de 4-6.

2.  Le procédé selon l'une quelconque des revendications précédentes dans lequel le pH de la précipitation est de 4,6-5.

**Fig. 1A**

**Fig. 1B**

**Fig. 2**

**Fig. 3**

**Fig. 4**

**Fig. 5**

Fig. 6A

Fig. 6B

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3455787 A

**Non-patent literature cited in the description**

- Purification and drying of bromelain. **DEVAKATE R V et al.** SEPARATION AND PURIFICATION TECHNOLOGY. ELSEVIER SCIENCE, vol. 64
- **ZATUL IFFAH MOHD ARSHAD et al.** Bromelain: an overview of industrial application and purification strategies. *APPLIED MICROBIOLOGY AND BIOTECHNOLOGY, DE,* vol. 98 (17
- **DÉBORA A CAMPOS et al.** Biological extraction of bromelain from pineapple byproducts. *Industrial Crops and Products Produção Aplicações e Mercado Biochemistry LWT Food Science and Technology,* 01 December 2015, 163-168
- **B. G. SMITH et al.** Polysaccharide Composition of Unlignified Cell Walls of Pineapple [Ananas comosus (L.) Merr.] Fruit. *PLANT PHYSIOLOGY,* 01 April 1995, vol. 107, 1399-1409
- **C.B. FABIAN et al.** Precipitation of rice bran protein using carrageenan and alginate. *LWT- FOOD SCIENCE AND TECHNOLOGY,* 01 March 2010, vol. 43 (2), 375-379